# EUROPEAN PATENT APPLICATION

(11) **EP 4 778 510 A1**
(43) Date of publication of application: **22.07.2026**
(21) Application number: 25152059.9
(22) Date of filing: 15.01.2025
(51) Int. Cl.: A61K 8/99, A61K 35/74, A61Q 19/00, A61K 35/00, A61Q 19/08

(54) **SKIN CARE COMPOSITION AND USES THEREOF**

(71) Applicant: S-Biomedic NV, 9052 Gent (BE)
(72) Inventor: The designation of the inventor has not yet been filed
(74) Representative: Eisenführ Speiser

(57) **Abstract**

The present invention generally relates to the field of skin care. More specifically, the invention relates to cosmetic skin care compositions comprising live bacteria, including at least one strain of the bacterial species *Cutibacterium acnes* (*C. acnes*). The products of the invention can be used for improving, maintaining and/or restoring skin microvascularization, thereby supporting the blood flow through the skin and contributing to a healthy and young appearance of the skin. The invention also relates to cosmetic uses of such skin care compositions and cosmetic methods that apply such skin care compositions.

## Description

### FIELD OF THE INVENTION

The present invention generally relates to the field of skin care. More specifically, the invention relates to cosmetic skin care compositions comprising live bacteria, including at least one strain of the bacterial species *Cutibacterium acnes (C. acnes).* The products of the invention can be used for improving, maintaining and/or restoring skin microvascularization, thereby supporting the blood flow through the skin and contributing to a healthy and young appearance of the skin. The invention also relates to cosmetic uses of such skin care compositions and cosmetic methods that apply such skin care compositions.

### BACKGROUND OF THE INVENTION

The human body is host to a highly complex and rich microbial community. These microorganisms are generally harmless and contribute to a healthy state by producing vitamins, cooperating with digesting food, or stimulating the immune system. The human microbiota mainly resides on the surface and in deep layers of skin, in the saliva and oral mucosa, in the conjunctiva, and in the gastrointestinal tracts.

The skin microbiota plays a major role in the barrier function of the skin and consequently also in human skin health and disease. The skin is colonized by a large number of microorganisms, most of them being beneficial or harmless. However, the skin microbiome has specific compositions in disease states of the skin that are different to healthy skin. Diseases such as acne vulgaris are associated with strong alterations of the microbiome. It has further been shown that major alterations occur in the skin microbiota during ageing of the skin. Although the skin microbial composition of healthy subjects has been found to remain largely stable over time during adulthood, age-related physiologic changes - particular alterations in sebum secretion and immune function and a decrease in sweat - may affect the skin microbiome of older individuals.

Alterations in the skin microbiota have thus been observed in both skin diseases, such as acne, as well as in the ageing skin. A key role herein seems to be for the facultative anaerobic gram-positive bacterium *Cutibacterium acnes* (C. acnes; formerly known as *Propionibacterium acnes). C. acnes* bacteria decompose the sebum to glycerine and fatty acids, thereby further inducing the production of sebum in the sebaceous glands and destroying the follicle walls in the skin. This results in inflammation of the skin and formation of pimples, pustules, nodules and cysts which often heal only with scarring.

On the other hand, *C. acnes* is one of the most abundant species of microorganisms on the skin, which suggests that it has co-evolved with humans and therefore, its presence may confer skin benefits. This hypothesis is strengthened by the exclusive niche that *C*. *acnes* inhabits - it is nearly the sole inhabitant of the sebaceous hair follicle. It has therefore been suggested that modulation of the skin microbiome in order to restore the microbiome into a healthy microbiome can be achieved by administering *C. acnes* bacteria to the skin. For example, WO 2016/172196 discloses a method of treating acne in a subject by administering a composition comprising one or more live *C. acnes* strains to the skin of the subject. Similarly, WO 2018/073651 discloses a composition for acne treatment comprising two or more different *C. acnes* strains, including *C. acnes* strain C3 and/or K8.

In addition, sufficient evidence nowadays exists that the normal, healthy epidermal skin is an anaerobic microenvironment (Resvani et al., 2011). The present invention is, amongst others, based on the insight that the application of *C*. *acnes* strains to the skin synergistically promotes the formation of anaerobic condition on the surface of the skin which in turn induces the production of Vascular Endothelial Growth Factor A (VEGFA) by skin cells, such as keratinocytes. VEGFA is a growth factor that is known to be important for the microvascularization of the skin.

In aged or photoaged skin, the microvascularization decreases for several reasons, amongst others, due to the reduced production of VEGFA by keratinocytes. The administering *C. acnes* bacteria to the skin therefore is a safe and efficient way to cosmetically rejuvenate the skin by restoring and/or increasing microvascularization.

### SUMMARY

Thus, in a first aspect the present invention relates to a skin care composition comprising live bacteria of at least one *C. acnes* strain for use in a method of improving, maintaining and/or improving or restoring cutaneous microvascularization.

In a second aspect, the present invention relates to the cosmetic use of a skin care composition comprising live bacteria of at least one *C. acnes* strain in improving, maintaining and/or restoring cutaneous microvascularization.

In a third aspect, the present invention relates to a cosmetic method for improving, maintaining and/or restoring cutaneous microvascularization in a subject, said method comprising the topical administration of a skin care composition comprising live bacteria of at least one *C. acnes* strain to the skin of the subject.

In a fourth aspect the present invention relates to a skin care composition comprising live bacteria of at least one *C. acnes* strain for use in a method of creating an anaerobic microenvironment on the surface of the epidermal skin of a subject.

In a fifth aspect, the present invention relates to the cosmetic use of a skin care composition comprising live bacteria of at least one *C. acnes* strain in creating an anaerobic microenvironment on the surface of the epidermal skin of a subject.

In a sixth aspect, the present invention relates to a cosmetic method for creating an anaerobic microenvironment on the surface of the epidermal skin of a subject, said method comprising the topical administration of a skin care composition comprising live bacteria of at least one *C. acnes* strain to the skin of the subject.

In a seventh aspect, the present invention relates to a skin care composition comprising live bacteria of at least one *C. acnes* strain for use in a method of improving, maintaining and/or improving or restoring cutaneous blood circulation.

In an eighth aspect, the present invention relates to the cosmetic use of a skin care composition comprising live bacteria of at least one *C. acnes* strain in improving, maintaining and/or restoring cutaneous blood circulation.

In a ninth aspect, the present invention relates to a cosmetic method for improving, maintaining and/or restoring cutaneous microvascularization in a subject, said method comprising the topical administration of a skin care composition comprising live bacteria of at least one *C. acnes* strain to the skin of the blood circulation.

### DETAILED DESCRIPTION OF THE INVENTION

With the present invention, the inventors surprisingly found that co-cultures of one or more *C. acnes* strain and keratinocytes synergistically increase the oxygen consumption in the cultures. The resulting anaerobicity leads to an increased production of angiogenic factors, such as VEGFA, by keratinocytes. Since the reduced production of VEGFA is a hallmark of skin aging, application of one or more *C. acnes* bacteria to the epidermal skin of a subject is helpful for creating an anaerobic microenvironment which in turn induces the production of VEGF which results in the improved microvascularization of the skin.

According to a first aspect of the invention, a skin care composition is provided for use in a method of improving, maintaining and/or restoring cutaneous microvascularization. The skin care composition comprises live bacteria. The bacteria in the skin care composition include at least one *C*. acnes strain, and preferably a *C*. acnes strain which, upon administration, contributes to restoring, maintaining and/or improving an anaerobic microenvironment on the surface of the epidermal skin.

In the context of the present invention, the term "microvascularization" and/or "microangio-genesis" refers to the development of new small blood vessels in an organ or body part. More particularly, the terms refer to the formation of new small blood vessels with an internal diameter of 100 microns or less, more preferably 90 microns or less, or 80 microns or less. Hence, the term "cutaneous microvascularization" refers to the development of new small blood vessels in the skin, wherein said blood vessels have an internal diameter of 100 microns or less, more preferably 90 microns or less, or 80 microns or less. As used herein, the terms "microvascularization of the skin" and "cutaneous microvascularization" may be used interchangeably.

As used herein, an anaerobic microenvironment of the epidermal skin is characterized by a partial oxygen pressure (PO2) in the epidermal skin that is below 60 mm Hg, preferably below 40 mm Hg. Vice versa, an aerobic microenvironment of the epidermal skin is characterized by a partial oxygen pressure (PO2) in the epidermal skin that is above 40 mm Hg, preferably above 60 mm Hg. Thus, in some embodiments, the impaired anaerobic microenvironment of the epidermal skin is characterized by a partial PO2 in the epidermal skin that is above 40 mm Hg, preferably above 60 mm Hg. In some embodiments, the anaerobic microenvironment of the epidermal skin is characterized by a partial PO2 in the epidermal skin that is below 60 mm Hg, preferably below 40 mm Hg.

In some embodiments, the restoring of the anaerobic microenvironment of the epidermal skin is characterized by bringing the partial PO2 in the epidermal skin to below 60 mm Hg, preferably to below 40 mm Hg.

As used herein, the "epidermal skin" refers to the superficial part of the skin. The human skin can be divided in two tissues, one superficial, the epidermal layer or epidermal skin, and the other deep tissue, the dermis or dermal skin. The natural human epidermal skin is mainly composed of three types of cells, keratinocytes, melanocytes and the Langerhans cells. The dermal skin provides the epidermal skin with solid support. It is also the nourishing element of the epidermal skin. The dermal skin mainly consists of fibroblasts and an extracellular matrix composed mainly of collagen and elastin. The dermal skin also comprises blood vessels and nerve fibres.

The oxygen partial pressure in the epidermal skin can for example be measured using O2-sensitive micro-electrodes, such as for example described in Wang et al. (2003).

In certain embodiments, the bacteria in the skin care composition may be lyophilized or spray-dried live bacteria. This means that viable bacteria have been subjected to a drying process that maintains their viability, but arrests or reduces their metabolic processes to a minimum. In lyophilized or spray-dried form, the bacteria can be stored for months or even years. Once they are applied to the skin, such as the human skin, the metabolism of the bacteria is reactivated such that they resume growth. They propagate on the skin surface and displace pathogenic bacterial strains, thereby recovering a diverse, healthy and balanced skin microbiome.

In one embodiment, the live *C. acnes* bacteria are present in spray-dried form. The principle of spray drying is based on the dispersion of a solution into fine droplets which are introduced into a flow of hot air. The solvent evaporates from the substrate droplets so that dry product clusters remain. Standard spray drying devices can be used, such as the Mini Spray Dryer B-290 from Büchi Labortechnik GmbH (Essen, Germany) or the Mobile Minor^{™} Spray Dryer from GEA (Berlin, Germany).

In one embodiment, the live *C. acnes* bacteria are present in freeze-dried or lyophilized form. Freeze drying or lyophilization is a process which includes freezing the product, reducing the pressure and adding heat to allow the frozen water in the material to sublimate. Various methods can be applied for freezing the product. For example, freezing can be achieved by using a standard freezer or a chilled bath. Cooling the product below its triple point ensures that sublimation will occur upon heating. To prevent the formation of large crystals that may damage the structure of the product to be dried, freezing is done rapidly. About 95% of the water in the product is removed when the frozen water sublimates. Most materials can be dried to 1-5% w/w residual moisture. Standard freeze drying devices can be used, such as the Lyovac^{™} devices from GEA (Berlin, Germany), the Gamma 2-20 Freeze dryer LCM-1 from Christ (Osterode am Harz, Germany), or the Christ Martin^{™} Alpha 1-2 Lyophilisator from Fisher Scientific GmbH (Schwerte, Germany).

In some embodiments, the skin care composition comprises at least one *C. acnes* strain, preferably at least one *C. acnes* strain each independently selected from the group consisting of the single locus sequence typing (SLST) type strains A1, D1, A5, C1, C3, H1, H2, H3, K1, K2, K4, K6, K8, K9, L1, and F4. In some embodiments, the skin care composition comprises lyophilized or spray dried live bacteria of at least one *C. acnes* strain each independently selected from the SLST type strains A1, A5, C3, D1, H1 and K2. In some embodiments, the skin care composition comprises lyophilized or spray dried live bacteria of at least one *C. acnes* strain each independently selected from the SLST type strains A1, D1, and H1. In some embodiments, the skin care composition comprises lyophilized or spray-dried live bacteria of at least one A1 strain. In some embodiments, the skin care composition comprises lyophilized or spray-dried live bacteria of at least one *C. acnes* SLST type D1 strain. In some embodiments, the skin care composition comprises lyophilized or spray-dried live bacteria of at least one *C. acnes* SLST type H1 strain.

In some embodiments, the concentration of each *C. acnes* strain, such as each lyophilized or spray-dried *C. acnes* strain, is at least 0.5% w/v of the skin care composition. In some embodiments, when more than one *C. acnes* strain is present in the skin care composition, each strain is at approximately equal concentrations within the composition. In some embodiments, one *C. acnes* strain is present at a higher concentration than the other one or more *C. acnes* strains within the composition.

The skin care composition of the present invention comprises (optionally lyophilized or spray-dried) live bacteria of at least one strain of the species *C. acnes.* In some preferred embodiments, the skin care composition may comprise two or more strains of the species *C. acnes.* For example, the skin care composition may comprise 2, 3, 4, 5, 6, 7, 8, 9, 10, 11, 12, 13, 14, 15, 16, 17, 18, 19, 20 or more than 20 strains of *C*. *acnes.* In some embodiments the skin care composition comprises 2, 3, 4 or 5 different strains of *C*. *acnes.*

The bacterial species *Cutibacterium acnes (C. acnes)* was formerly known as *Propionibacterium acnes (P. acnes).* Based on the results from biochemical and genomic studies, the species was taxonomically reclassified in 2016. *C. acnes* is a Gram-positive, anaerobic, rod-shaped bacterium which is known to be involved in the development of acne and other pathological conditions. *C. acnes* is largely commensal and is an abundant colonizer of human skin thriving in the nutritious environment of sebum rich skin. The bacterium causes no symptoms in the majority of carriers, but certain strains have a suggested pro-inflammatory role in acne vulgaris. Moreover, due to its biofilm-forming capacity, the bacterium is frequently isolated from various orthopaedic implant-associated infections.

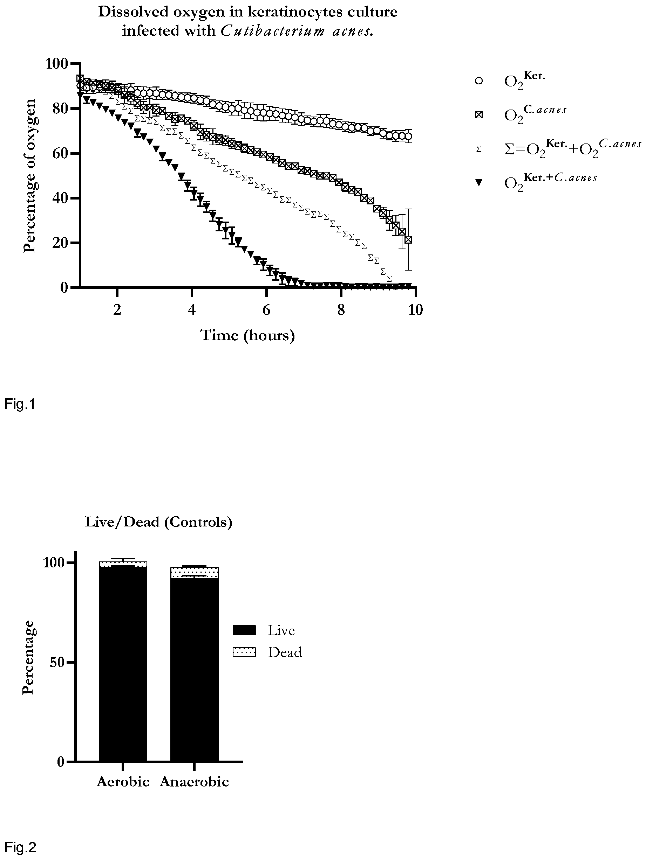

Studies by Johnson and Cummins (1972) first revealed two distinct phenotypes of *Propionibacterium acnes,* known as types I and II, that could be distinguished based on serological agglutination tests and cell wall sugar analysis. Subsequent studies, involving among others sequence analysis of the recA gene demonstrated that P. *acnes* comprises four highly distinct evolutionary lineages, known as type or clade IA, IB, II and II that display differences in inflammatory properties, production of virulence determinants and association with various conditions (McDowell at al. 2005; McDowell at al. 2008; Valanne et al. 2005; Lodes et al. 2006). Type IA has been further subdivided into subtypes IA1 and IA2, and type IC has been identified using a multilocus sequence typing scheme (eMLST) based on six housekeeping genes and two putative virulence genes (McDowell et al. 2012). Scholz et al. (2014) later developed a single-locus sequence typing (SLST) scheme for P. *acnes* strains, involving PCR amplification and DNA sequencing of the target locus PPA2385. A publicly available *P. acnes* database and SLST allocation tool associated with the SLST scheme described by Scholz et al. is available online at medbac.dk/slst/pacnes. Exemplary SLST types for *P*. *acnes* strains include SLST types A1 to A24, B1, C1 to C4, D1 to D3, E1 to E9, F1 to F10, G1, H1 to H5, K1 to K14, and L1 to L6. Other *P*. *acnes* strain typing schemes are known as MLST9, MLST8, and ribotype schemes, reviewed in Scholz et al., especially in Figure 1. Where SLST types are referred to in this specification, the types according to the SLST typing scheme of Scholz et al., are meant. As further guidance but without limitation, Table 1 on p. 20-31 of WO 2018/073651, incorporated by reference herein, lists several allelic sequences of the target locus PPA2385 used in Scholz et al.'s SLST to identify SLST types of *P*. *acnes* strains.

Illustrative but non-limiting strains of *C*. *acnes* useful herein can be obtained from public microorganism collections maintained for example by Leibniz Institute DSMZ-German Collection of Microorganisms and Cell Cultures (Inhoffenstr. 7B, D-38124 Braunschweig, Germany, https:www.dsmz.de/catalogues.html), or by American Type Culture Collection (ATCC) 10801 University Blvd. Manassas, Virginia 20110-2209, USA) or by European Collection of Cell Cultures (ECACC) (Heath Protection Agency - Porton Down Salisbury, Wilthsire SP4 0JG, United Kingdom), including *C. acnes* strain KPA171202 (DSMZ acc. No. DSM-16379), *C. acnes* subsp. defendens strain (acc. no. ATCC 11828), *C. acnes* strain (acc. no. ATCC 6919), *C. acnes* subsp. elongatum strain (ECACC acc. no. NCTC 13655), and others.

Further, *C. acnes* strains of virtually any type or subtype useful herein can be readily sampled and isolated from the skin of adult humans, preferably healthy adults, or from *C. acnes* biofilms on infected orthopaedic prostheses. Any available protocol for isolation, culture and expansion of *C*. *acnes* can be adopted. Hence, in certain embodiments, a composition can be formed by isolating and culturing one or more *C. acnes* from a donor subject, particularly a donor subject not having oxidative stress-associated skin disease and preferably a donor subject having overall healthy skin, and combining it with other one or more carriers to form the composition.

By means of example and without limitation, one suitable protocol for obtaining *C. acnes* isolates from the skin was described in Holmberg et al. (2009). Therein, an area of 3x3 cm of the forehead of a human subject was swabbed with a cotton-tipped swab, moist with sterile physiologic saline, and plated on blood agar (LabM Ltd, Heywood, Bury, United Kingdom) containing horse blood (4%). The plates were incubated under anaerobic conditions (10% H₂, 10% CO₂, 80% N₂) at 37°C for 72 hours. Isolates were characterized as *C*. *acnes* using routine microbiological criteria, including characteristic macroscopic appearance (typical small colonies), Gram-staining characteristics, and the production of catalase and indole. Isolates were frozen at-80°C in glycerol (50% v/v). The bacteria could be further cultivated in Bacto Brain Heart Infusion broth (BHI; Becton and Dickinson, Sparks, MD, USA) supplemented with glucose (0.5%) and kept in an anaerobic chamber for 72h at 37°C. When performed on48 healthy individuals, this yielded 48 *C. acnes* isolates belonging to types IA, IB, II (see Table 2 of Homberg, et al., incorporated by reference herein).

*C. acnes* cells can be further cultured and expanded in a variety of media, including rich or minimal media, preferably rich media such as those mentioned above. As would be understood by one of ordinary skill in the art, routine optimization would allow for use of a variety of types of media. Media can be supplemented with various additional components, including sugar sources. Some non-limiting examples of supplemental components include glucose, amino acids, vitamins, lipids, glycerol, and ATCC Trace Mineral Supplement. Similarly, other aspects of the medium, and growth conditions of the cells of the invention can be optimized through routine experimentation. For example, pH, temperature, and concentration of components within the compositions are non-limiting examples of factors which can be optimized. Liquid and/or solid cultures used to grow *C. acnes* cells can be housed in any of the culture vessels known and used in the art. In some embodiments, the C. acnes strains are grown in batches. In some embodiments, the bacterial strains are grown in fermenters. In some embodiments, the compositions comprising the bacterial strains are packaged. In certain embodiments, compositions comprising the bacterial strains are packaged in enteral syringes or sachets. In certain embodiments, the bacterial strains may be freeze-dried.

It was previously reported that some strains of *C*. *acnes* are associated with, contribute to or are causative of acne, while other strains show no such connection to acne (Fitz-Gibbon et al. 2013; Lomholt et al., 2010). These authors also characterized certain genetic and metabolic determinants of the ability of *C*. *acnes* to contribute to acne. Similarly, WO 2018/073651 described metabolic assays by which pathogenic and non-pathogenic strains of *C*. *acnes* could be identified and selected based on their expression of active linoleic acid isomerase which specifically converts cis-9, cis-12 linoleic acid into trans-10, cis-12 linoleic acid. Accordingly, in certain embodiments, the *C*. *acnes* strain orstrains as intended herein are not associated with, do not contribute to and are not causative of acne. Such *C*. *acnes* strains can be readily isolated from healthy human skin (as opposed to human skin affected by acne).

The term "strain" is well-understood as a low-level taxonomic rank used within a species. In microbiology, a strain may be typically defined operatively - a strain is made up to the descendants of a single isolation in pure culture and usually is made up of a succession of cultures ultimately derived from an initial single colony; or alternatively, as an isolate or group of isolates that can be distinguished from other isolates of the same genus and species by phenotypic characteristics or genotypic characteristics or both. As used in the practice of the present invention, the term "strain" may be synonymous with the operative terms "isolate" or "clone".

The term "live" as used herein is synonymous with "viable" and refers to any living intact state of a microorganism, such as active growth or dormancy, from which state it can multiply and/or reproduce itself in a medium capable of supporting the growth of the microorganism. Typically, substantially all *C. acnes* bacteria comprised by the compositions intended herein may be live or viable. For example, at least 50%, preferably at least 60%, more preferably at least 75%, still more preferably at least 90%, such as at least 95%, 96%, 97%, 98%, 99% or 100% of the bacteria in the composition are viable, such as capable of forming colonies when plated on a suitable solid medium.

*C. acnes* strains occur on the skin of most people. *C. acnes* strains can be pathogenic or non-pathogenic. As used herein, "pathogenic" *C. acnes* strains are strains that are associated with acne. Assays for the identification and selection of pathogenic and non-pathogenic C. acnes strains are described in WO 2018/073651.

*C. acnes* has been shown to comprise several distinct, major phylogenetic groups classified as types I, II and III, with the major type I clade being further divided into sub-clades known as types IA, IB and IC (Lomholt and Kilian, 2010). Sub-clade IA has been further subdivided into IA1 and IA2 (McDowell et al., 2012). Preferably, the at least one strain of the species *C. acnes* is a non-pathogenic strain of *C*. *acnes.* A genetic analysis of *C*. *acnes* strains revealed that strains which are non-pathogenic and not associated with acne are mainly members of (i) clade I, sub-clade IA, (ii) clade I, sub-clade IB and (ii) clade II. *C. acnes* strains belonging to these claims may be preferably employed herein. Accordingly, in one embodiment of the invention, the at least one strain of the species *C. acnes* belongs either to one of sub-clades IA, IB of clade I or to clade II. In one embodiment, the at least one strain of the species *C. acnes* belongs to sub-clade IA. In another embodiment, the at least one strain of the species *C. acnes* belongs to sub-clade IB. In yet another embodiment, the at least one strain of the species *C. acnes* belongs to clade II. If more than one strain is used in the skin care composition or kit of parts of the present invention, it is preferred that strains from different clades or sub-clades are mixed with each other. For example, in one embodiment, the skin care composition or kit of parts comprises at least one strain from sub-clade IA and at least one strain from sub-clade IB. In another embodiment, the skin care composition or kit of parts comprises at least one strain from sub-clade IA and at least one strain from clade II. In yet another embodiment, the skin care composition comprises at least one strain from sub-clade IB and at least one strain from clade II. In some embodiments, live bacteria of the *C. acnes* strain with SLST type A1 belong to sub-clade IA. In some embodiments, live bacteria of the *C. acnes* strain with SLST type D1 belong to sub-clade IA. In some embodiments, live bacteria of the *C. acnes* strain with SLST type H1 belong to sub-clade IB.

In other embodiments, the skin care composition may comprise a mixture of *C*. *acnes* strains that include one or more clade I strains and one or more clade II strains. While clade II strains, as indicated above, may be less pathogenic than clade I strains, these strains can also be slower-growing than clade I strains, and less likely to be able to colonize the skin on their own. Accordingly, it may in some embodiments be advantageous that the skin care composition or kit of parts includes a mixture of strains that include both clade I and clade II strains which allow for comparatively improved colonization of the skin by clade II strains.

Non-limiting examples for non-pathogenic strains of *C*. *acnes* include, but are not limited to the SLST type strains A1, D1, A5, C1, C3, H1, H2, H3, K1, K2, K4, K6, K8, K9, L1 and F4. In a preferred embodiment, the skin care composition of the present invention includes at least one strain selected from the SLST type strains A1, A5, C3, D1, H1 and K2. It is particularly preferred that the skin care composition of the present invention includes at least one SLST type A1 strain and/or at least one SLST type D1 strain and/or at least one SLST type H1 strain. In one embodiment, the skin care composition of the present invention includes at least one SLST A1 strain, and more preferably two or more SLST type A1 strains, such as, 3, 4, 5, 6, 7, 8, 9 or 10 SLST A1 strains. In another embodiment, the skin care composition of the present invention includes at least one SLST D1 strain, and more preferably two or more SLST type D1 strains, such as, 3, 4, 5, 6, 7, 8, 9 or 10 SLST D1 strains. In yet another embodiment, the skin care composition of the present invention includes at least one SLST H1 strain, and more preferably two or more SLST type H1 strains, such as 3, 4, 5, 6, 7, 8, 9 or 10 SLST H1 strains. In some other embodiments, the skin care composition of the present invention includes a combination of two or more of the SLST type A1, D1, and H1 strains. Such as for example, in an embodiment the skin care composition of the present invention includes at least one SLST type H1 strain in combination with at least one SLST D1 strain. Or, in some embodiments, the skin care composition of the present invention includes at least one SLST type H1 strain in combination with at least one SLST A1 strain and in combination with at least one SLST type D1 strain. In some other embodiments, the skin care composition of the present invention includes at least one SLST type H1 strain in combination with at least one SLST A1 strain.

In a particularly preferred embodiment, the skin care composition of the present invention comprises at least one *C. acnes* strain selected from the group consisting of the SLST type A1 strain, SLST type H1 strain, and SLST type D1 strain. In some other preferred embodiment, the skin care composition comprises at least one *C. acnes* strain selected from the group consisting of the SLST type A1 strain, SLST type H1 strain, and SLST type D1 strain.

As indicated above, the strains designation referred to herein is based on the single-locus sequence typing (SLST) scheme described in Scholz et al., 2014 using locus PPA2385 as the SLST target sequence. The SLST types can be interrogated using the following primers (Table 1):

**Table 1: Primer sequences used to type bacterial colonies**

| SEQ ID NO: | Description | Sequence |
|---|---|---|
| 1 | Forward primer | CAGCGGCGCTGCTAAGAACTT |
| 2 | Reverse primer | CCGGCTGGCAAATGAGGCAT |
| 3 | SLST-Adapter -FW | TCGTCGGCAGCGTCAGATGTGTATAAGAGACAGCAGCGGCGCTGC TAAGAACTT |
| 4 | SLST-Adapter -RV | GTCTCGTGGGCTCGGAGATGTGTATAAGAGACAGCCGGCTGGCAA ATGAGGCAT |

The sequences of the PPA2385 locus of the different strains identified by Scholz are listed as SEQ ID NO: 1-76 in Table 1 on p. 20-31 of WO 2018/073651, incorporated by reference herein.

Accordingly, by means of illustration, a "SLST type A1" strain is a strain that comprises in its genome a sequence of the PPA2385 locus which is 100% identical to the following sequence:

GTTGCACACCAGGGGGTCAACTTGGCGTCCTCAGTTCAAAATTGATTCAAACTAACAGTTCCATGT
CGGGAAACAGCACCAGGAAGCTCGTGACATATCGTCTTTCATTGCGAGAAACATCTTACTTATGTA
CATTTCTAAGCTATAGCGTCTACCCTTGTCAGACCCAGGACGATGGGTGTCACATCTCCTTTCTAG
TCAACCTAAGAGAGGAGGAAATGCCGCGATATATGTTCCACCCTGTCATCACGAAGGCCACCACAA
TCTATCCCAGAACAGCCGGCACTTCACTCACGATGCCCCGATGCTGGATTCCTATTGTCGCCCTTA

TTAGGGCAAGCGGTGCCAGTAGCAGAATATGTCACCTCAACAACTCGATCCACCCCTGCCCATTAC
ATGGGTAACATATCCATGGAGGTTCGATGTATACTCGAGGATACAGTCGTCCATCACGCCCGCCTA
CATACCCATTACATCAGCATAG (SEQ ID NO: 5).

A "SLST type D1" strain is a strain that comprises in its genome a sequence of the PPA2385 locus which is 100% identical to the following sequence:

GTTGCACACCAGGGGGTCAACTTGGCGTCCTCAGTTCAAAATTGATTCAAACTAACAGTTCCATGT
CGGGAAACAGCACCAGGAAACTCGTGACATATCGTCTTTCATTGCGAGAAACATCTTACTTATGTA
CATTTCTAAGCTATAGCGTCTACCCTTGTCAGACCCAGGACGATGGGTGTCACATCCCCTTTCTAG
TCAACCTAAGAGAGGAGGAAATGCCGCGATATATGTTCCGCCCTGTCATCACGAAGACCACCACAA
TCTATCCCAGAACAGCCGGCACTTCACTCACGATGCCCCGATGCTGGATTCCTATTGTCGCCCTTA
TTAGGGCAAGCGGTGCCAGTAGCAGAATATGTCACCTCAACAACTCGATCCACCCCTGCCCATTAC
ATGGGTAACATATCCATGGAGGTTCGATGTATACTTGAGGATACAGTCGTCCATCACGCCCACCTA
CATACCCATTACATCAGCATAG (SEQ ID NO: 6).

A "SLST type H1" strain is a strain that comprises in its genome a sequence of the PPA2385 locus which is 100% identical to the following sequence:

GTTGCACACCAGGGGGTCAACTTGGCGTCCTCAGTTCAAAATTGATTCAAACTAACAGTTCCATGT
CGGGAAACAGCACCAGGAAGCTCGTGACATATCGTCTTTCATTGCGAGAAACATCTTACTTATGTA
CATTTCTAAGCTATATCGTCTACCCTTGTCAGACCCAGGACGATGGATGTCACATCCCCTTTCTAG
TCAACCTAAGAGAGGAGGAAATGCCGCGATATATGTTCCACCCTGTCATCACGAAGGCCACCACAA
TCTATCCCAGAACAGCCGGCACTTCACTCACGATGCCCCGATGCTGGATTCCTATTGTCGCCCTTA
TTAGGGCAAGCGGTGCCAGTAGCAGAATATGTCACCTCAACAACTCGATCCACCCCTGCCCATTAC
ATGGGTAACATATCCATGGAGGTTCGATGTATATTCGAGGATACAGTCGTCCATCACGCCCGCCTA
CATACCCATTACATCAGCATAG (SEQ ID NO: 7).

The SLST scheme is also described in more detail in WO 2018/073651. Sequence identification of the PPA2385 locus can be performed as described in WO 2018/073651 by PCR amplification and DNA sequencing using the nucleotide primer set forth as SEQ ID NO: 77-82 in Table 2 on p. 31 of WO 2018/073651.

The composition of the present invention may include both pathogenic strains and non-pathogenic strains of *C*. *acnes.* However, in a preferred embodiment of the invention, the skin care composition comprises exclusively non-pathogenic strains of *C*. *acnes.* It is particularly preferred that the skin care composition of the invention does not include a ribotype 6 (RT6) strain of *C*. *acnes.* The ribotype classification system is based on difference in the 16S rDNA sequence between different strains of *C*. *acnes.* The ribotype system is explained, for example, in Fitz-Gibbon et al. 2013. It is further particularly preferred that the skin care composition of the invention does not include a Phylotype III strain of C. acnes.

*C. acnes* strains are normally able to produce the signalling molecule trans-10, cis-12 linoleic acid from its precursor molecule linoleic acid, the latter of which is naturally present in the sebum. Trans-10, cis-12 linoleic acid is thought to stimulate sebum production and secretion which is important for *C*. *acnes* colonization of the skin. In this way, trans-1 0, cis-12 linoleic acid promotes the onset of acne. Dependent on the skin of a subject, it may be favourable to either reduce or increase sebum production. For example, it may be useful to reduce sebum production in a skin of a subject suffering from acne or oily skin. To the contrary, it may be useful to increase sebum production in the skin of a subject suffering from dry skin, or aged or photoaged skin.

In one embodiment, the one or more *C. acnes* strains of the present invention are hence selected based on its ability to produce trans-10, cis-12 linoleic acid. In one preferred embodiment, strains that produce low levels of trans-10, cis-12 linoleic acid are selected for use against acne or oily skin. Without wishing to be bound by theory, these strains are thought to reduce sebum production, which is useful for preventing or reducing the symptoms of acne or oily skin. SLST type strains C3, C1, F4, A5, K1, K2, K8 and L1 produce only low amounts of trans-10, cis-12 linoleic acid. In another preferred embodiment, strains that produce high levels of trans-10, cis-12 linoleic acid are selected for use against dry skin. Without wishing to be bound by theory, these strains are thought to increase sebum production, which is useful for preventing or reducing the symptoms of dry skin (which is beneficial to reduce signs of aging). SLST type strain A1 produces high amounts of trans-10, cis-12 linoleic acid. In one embodiment, the one or more C. acnes strains to be administered to the skin area have been isolated from the skin microbiome of a donor subject. The subject may not be afflicted with acne or oily skin or may suffer from mild, moderate or severe acne. In another embodiment, the strains that have been isolated from the skin microbiome of a donor subject are non-pathogenic strains.

The one or more live *C. acnes* may also comprise one or more genetically modified strains of *C*. *acnes.* In another embodiment, one or more genetically *C. acnes* strain may be combined with one or more naturally occurring strains of *C*. *acnes.* The genetically modified strains have preferably been modified to produce lower or higher amounts of trans-10, cis-12 linoleic acid. The production of trans-10, cis-12 linoleic acid can be detected as described in US Patent 6,743,609 or by other commonly known methods, such as FAME (fatty acid methyl esters) or gas chromatography. In certain other embodiments, the one or more live *C. acnes* strains may only comprise naturally occurring strains of *C*. *acnes,* i.e., only *C*. *acnes* strain(s) that have not been genetically modified by man.

In some embodiments, each of the *C. acnes* strains in the skin care composition is present in an amount of 1.0 x 10⁴-1.0 x 10¹¹ colony forming units per ml (CFU/ml), preferably 1.0 x 10⁴-1.0 x 10⁹, even more preferably 1.0 × 10⁶-1.0 x 10⁹ CFU/ml such as 1.0 x 10⁷-1.0 x 10⁹ CFU/ml, or 1.0 x 10⁸-1.0 x 10⁹ CFU/ml. For example, the at least one *C. acnes* strain may be present in an amount of at least 1.0 x 10⁵ CFU/ml, preferably at least 1.0 x 10⁶ CFU/ml, more preferably at least 1.0 x 10⁷ CFU/ml, such as at least 1.0 x 10⁸ CFU/ml, or at least 1.0 x 10⁹ CFU/ml of the skin care composition.

In some embodiments, each of the *C. acnes* strains that is present in the skin care composition is present in an amount of 1.0× 10⁴-1.0x 10¹¹ CFU/ml, preferably 1.0 x 10⁴-1.0 x 10⁹ CFU/ml, more preferably 1.0 x 10⁶-1.0 x 10⁹ CFU/ml and even more preferably 1.0 x 10⁷-1.0 × 10⁹ CFU/ml, or 1.0 x 10⁸-1.0 × 10⁹ CFU/ml. For example, each of the *C. acnes* strains may be present in an amount of at least 1.0 x 10⁵ CFU/ml, preferably at least 1.0 x 10⁶ CFU/ml, more preferably at least 1.0 x 10⁷ CFU/ml, such as at least 1.0 x 10⁸ CFU/ml, or at least 1.0 × 10⁹ CFU/ml of the composition. For example, if the composition of the present invention comprises one SLST type H1 strain and one SLST type K8 strain, each of these strains may be present in an amount of 1.0 x 10⁴-1.0 x 10⁹ CFU/ml, such as 1.0 x 10⁹ CFU/ml, 2.0 x 10⁹ CFU/ml, 3.0 x 10⁹ CFU/ml, 4.0 x 10⁹ CFU/ml, 5.0 x 10⁹ CFU/ml, 6.0 x 10⁹ CFU/ml, 7.0 x 10⁹ CFU/ml, 8.0 x 10⁹ CFU/ml, or 9.0 x 10⁹ CFU/ml.

In some embodiments, the overall amount of (optionally lyophilized or spray-dried) bacteria in the composition is 1.0 × 10⁴-1.0 × 10¹¹ CFU/ml, such as 1.0 × 10⁴-1.0 × 10⁹ CFU/ml, more preferably 1.0 x 10⁵-1.0 x 10¹⁰ CFU/ml, and even more preferably 1.0 x 10⁷-1.0 x 10¹⁰ CFU/ml, or 1.0 x 10⁸-1.0 x 10⁹ CFU/ml. For example, the bacteria may be collectively present in the composition in an amount of at least 1.0 x 10⁵ CFU/ml, preferably at least 1.0 x 10⁶ CFU/ml, more preferably at least 1.0 x 10⁷ CFU/ml, such as at least 1.0 x 10⁸ CFU/ml, at least 1.0 x 10⁹ CFU/ml, or at least 1.0 x 10¹⁰ CFU/ml of the composition. It is particularly preferred that the bacteria are collectively present in the composition in an amount of at least 1.0 x 10¹⁰ CFU/ml, 2.0 x 10¹⁰ CFU/ml, 3.0 x 10¹⁰ CFU/ml, 4.0 x 10¹⁰ CFU/ml, 5.0 x 10¹⁰ CFU/ml, 6.0 x 10¹⁰ CFU/ml, 7.0 x 10¹⁰ CFU/ml, 8.0 x 10¹⁰ CFU/ml, or 9.0 x 10¹⁰ CFU/ml of the composition. One of ordinary skill in the art will be readily able to determine the amount of bacteria in the composition.

In some embodiments, the skin care composition is configured for topical administration to the skin. In some embodiments, the skin care composition as taught herein is a gel, cream, ointment, lotion, drops, spray, including aerosol spray, foam or powder.

In some embodiments, the skin care composition is an aqueous preparation, such as a gel. Aqueous preparations as intended herein encompass aqueous solutions, as well as aqueous dispersions. In one embodiment, the composition is an oil-in-water emulsion. If the composition contains an oil phase, e.g. when using an oil-in-water emulsion, it is preferred that the oil phase contains triglycerides and/or octyldodecanol. In addition, the oil phase may contain one or more oils selected from the group of lecithin, olive oil, sunflower oil, jojoba oil, soya oil, peanut oil, rapeseed oil, almond oil, palm oil, coconut oil, castor oil, wheat germ oil, grape seed oil, safflower oil, evening primrose oil, macadamia nut oil and the like.

In some embodiments, the composition comprising one or more live *C. acnes* strain is a water-in-oil emulsion.

In other embodiments, the composition comprising one or more live *C. acnes* strain a can be an ointment or a cream. In the context of the present invention, an ointment is a semisolid preparation containing an ointment base and optionally one or more active agents, more specific the one or more live *C. acnes* strain. Examples of suitable ointment bases include hydrocarbon base (e.g., petrolatum, white petrolatum, yellow ointment, and mineral oil), absorption bases (e.g., hydrophilic petrolatum, anhydrous lanolin, lanolin, and cold cream), water-removable bases (e.g., polyethylene glycol ointments).

In still other embodiments, the composition comprising one or more live *C. acnes* strain can be a lotion. A lotion is a low- to medium-viscosity liquid formulation. A lotion can contain finely powdered substances that are insoluble in the dispersion medium through the use of suspending agents and dispersing agents. Alternatively, a lotion can have the dispersed phase liquid substances that are immiscible with the vehicle and are usually dispersed by means of emulsifying agents or other suitable stabilizers.

In certain embodiments, the compositions may be comprised by a mask, pad, patch, a two-chamber device (two-component dispensing system), or make-up. The present compositions may typically be intended as "leave-on" compositions. By means of an example and without limitation, the composition may comprise one or more components provided in one container, such as one chamber of a multiple-chamber device (e.g., a two-chamber dispensing system), and one or more components provided in another container, such as another chamber of the multiple-chamber device. Such arrangement allows the consumer or practitionerto admix the components of the composition shortly before use. For example, the composition may comprise the one or more live *C. acnes* strain or strains in one container, such as one chamber of a multiple-chamber device (e.g., a two-chamber dispensing system), and a composition comprising one or more other compounds, in another container, such as another chamber of the multiple-chamber device, to be admixed by the consumer or practitioner before use. Multiple-chamber (such as two- or dual-chamber) dispensers, such as bottles, tubes, jars, pumps, etc. are generally known and commercially available. By means of an example and not limitation, WO 2012/153206, WO 2017/168263, and WO 2018/060799 by Bormioli Rocco SPA (Fidenza, Italy) describe systems, marketed inter alia as the New Shaker^{®} single-dose dispenser, comprising a container and a closure capsule for closing the container, wherein the closure capsule includes an enclosure defined at least partly by a frangible bottom or mouth. Changing the configuration of the frangible bottom or mouth from intact to open allows to mix the previously separated contents of the container and the enclosure directly before use. In certain embodiments, the one or more live *C. acnes* strain or strains may be in a lyophilised form. Typically, such form is itself a composition comprising the one or more live *C. acnes* strain or strains and components of a suitable bacterial lyophilisation medium.

In some embodiments, the one or more live *C. acnes* strain are provided as a lyophilizate which is suspended in oil and is provided in a first container. A second container contains a composition which can be a carrier gel, cream, ointment or lotion. The content of both containers is mixed in the hands of the user immediately prior to applying the mixture to the face.

The skin care composition as taught herein may further comprise one or more carriers or excipients, which can broadly include any and all solvents, diluents, buffers (such as, e.g., neutral buffered saline, phosphate buffered saline, or optionally Tris-HCl, acetate or phosphate buffers), solubilizers (such as, e.g., Tween^{®} 80, Polysorbate 80), colloids, dispersion media, vehicles, fillers, chelating agents (such as, e.g., EDTA or glutathione), amino acids (such as, e.g., glycine), proteins, disintegrants, binders, lubricants, wetting agents, emulsifiers, sweeteners, colorants, flavourings, aromatizers, thickeners, agents for achieving a depot effect, coatings, antifungal agents, preservatives (such as, e.g., Thimerosal^{™} , benzyl alcohol), antioxidants (such as, e.g., ascorbic acid, sodium metabisulfite), tonicity controlling agents, absorption delaying agents, adjuvants, bulking agents (such as, e.g., lactose, mannitol) and the like. The use of such media and agents for the formulation of pharmaceutical and cosmetic compositions is well known in the art. In certain embodiments, the compositions may further comprise hydrators, exfoliants, humectants, emollients, and/or synthetic surfactants.

In a preferred embodiment, the skin care composition further comprises an emollient. As used herein, an emollient is a compound that moisturizes and/or softens the skin. Emollients normally reduce the roughness, cracking and/or irritation of the skin by penetrating into deeper layers of the skin. Emollients commonly used in skin care products comprise plant oils, like sesame oil, coconut oil, olive oil, almond oil, macadamia nut oil, cottonseed oil, or peanut oil, silicone oils, like dimethylpolysiloxane and cyclomethicone, fatty acids, and fatty alcohol ethers. The emollient can for example be selected from dicaprylyl carbonate, ethylhexyl cocoate, and mixtures thereof. When dicaprylyl carbonate is used as an emollient, it is preferably used in the final skin care composition in an amount of 0.05 to 25.0 wt%, more preferably 2.0 to 20.0 wt%, and more preferably 5.0 to 10.0 wt% or 7.5 to 10.0 wt%, relative to the total weight (w/w) of the composition. Stated differently, the amount of dicaprylyl carbonate in the skin care composition of the invention may be at least 0.05 wt%, at least 0.1 wt%, at least 0.25 wt%, at least 0.5 wt%, at least 0.75 wt%, at least 1.0 wt%, at least 1.25 wt%, at least 1.5 wt%, at least 1.75 wt%, at least 2.0 wt%, at least 2.5 wt%, at least 3 wt%, at least 4.0 wt%, at least 5.0 wt%, at least 6.0 wt%, at least 7.0 wt%, at least 8.0 wt%, or at least 9.0 wt%, relative to the total weight (w/w) of the skin care composition. When ethylhexyl cocoate is used as an emollient, it is preferably used in the final skin care composition in an amount of 0.05 to 25.0 wt%, more preferably 2.0 to 20.0 wt%, and more preferably 5.0 to 10.0 wt% or 7.5 to 10.0 wt%, relative to the total weight (w/w) of the composition. Stated differently, the amount of ethylhexyl cocoate in the skin care composition of the invention may be at least 0.05 wt%, at least 0.1 wt%, at least 0.25 wt%, at least 0.5 wt%, at least 0.75 wt%, at least 1.0 wt%, at least 1.25 wt%, at least 1.5 wt%, at least 1.75 wt%, at least 2.0 wt%, at least 2.5 wt%, at least 3 wt%, at least 4.0 wt%, at least 5.0 wt%, at least 6.0 wt%, at least 7.0 wt%, at least 8.0 wt%, or at least 9.0 wt%, relative to the total weight (w/w) of the skin care composition. When dicaprylyl carbonate and ethylhexyl cocoate are used in combination with each other as emollients, it is preferred that the overall amount of emollient is at least 0.05 wt%, but does not exceed 20.0 wt%, more preferably does not exceed 15.0 wt% or 10.0 wt% relative to the total weight (w/w) of the composition.

In another embodiment, the skin care composition further comprises a thickener. Thickeners are compounds that increase the viscosity of a cosmetic or pharmaceutical formulation. Thickeners are often polymers that absorb water and swell up, thereby making the composition more viscous. Thickeners commonly used in skin care products comprise bean gum, xanthan gum, gelatine, Carbauba wax, and stearic acid.

In a related embodiment, the skin care composition or the kit of parts further comprises a pH adjuster. Since the composition of the invention is used on the human skin, it will preferably have a neutral or slightly acidic pH to make it more compatible with the acidic environment of the skin. The composition may have a pH in the range from about 2.5 to about 7.5, preferably from about 4.0 to about 7.0, and more preferably from about 6.0 to about 7.5, preferably from about 4.0 to about 7.0, and more preferably from about 6.0 to about 7.0. An acidic pH in a cosmetic or pharmaceutical formulation can be normally achieved by adding an acid, such as formic acid, acetic acid, butyric acid, valeric acid, caproic acid, enanthic acid, or caprylic acid. Preferably, the pH adjuster is a citric acid/citrate buffer, which tends to display less interference with or can even promote the ability of the bacteria in the composition to grow and replicate after administration to the skin.

In yet another related embodiment, the skin care composition further comprises a filler. As used herein, a filler is a compound that aids in making the skin care composition more homogeneous by uniformly dispersing in the composition. Fillers are used to improve the sensory properties of the skin. Depending on the filler material, the end product may confer a silky, dry, smooth, or powdery skin feel. Fillers that can be used in the present invention can for example be distarch phosphate, tapioca starch, and mixtures thereof.

In another embodiment, the skin care composition further comprises a solubilizer. As used herein, a solubilizer is a compound that aids in the solubilization of hydrophobic substances in aqueous and alcohol formulations. For example, a solubilizer may render feasible the solubilization of perfume oils and other hydrophobic substances, such as vitamins, into aqueous composition. A particularly preferred solubilizer is polyethylene glycol 40 (PEG-40) castor oil, which tends to display less interference with or can even promote the ability of the bacteria in the composition to grow and replicate after administration to the skin.

Surfactants can be selected from the group consisting of anionic, cationic, amphoteric and alkylglycosidic surface active agents. Suitable anionic surfactants include, for example, alkyl and alkyl ether sulfates (such as sodium cocoalkyl triethylene glycol ether sulfate); water-soluble salts, phosphates such as monoalkyl, dialkyl, and trialkylphosphate salts formed by the reaction of phosphorous pentoxide with monohydric branched or unbranched alcohols having from about 8 to about 24 carbon atoms (such as mono or dilaurylphosphate); the reaction products of fatty acids esterified with isethionic acid and neutralized with an alkaline reagent; sulfonated fatty acids (such as alpha sulphonated coconut fatty acid and lauryl methyl ester); acyl isethionates (such as ammonium cocoyl isethionate, sodium cocoyl isethionate, sodium lauroyl isethionate); acyl glutamates (such as sodium lauroyl glutamate and sodium cocoyl glutamate); sulfocuccinate salts (such as disodium N-octadecyl-sulfosuccinamate and sodium dioctyl sulfosuccinate); carboxylates, including alkyl ether carboxylates (such as sodium laureth carboxylate); acyl lactylates (such as sodium cocoyl lactylate); alanoyl sarcosinates (such as sodium lauroyl sarcosinate, sodium cocoyl sarcosinate, and ammonium lauroyl sarcosinate); alkylglyceryl ether sulfonates (such as sodium cocoglyceryl ether sulfonate); and olefin sulfonates (such as sodium C14-16 olefin sulfonates). Suitable non-ionic surfactants include, but are not limited to, for example, compounds produced by the condensation of alkylene oxide with an organic hydrophobic compound which can be either aliphatic, alicyclic or aromatic in structure. Non-ionic surfactants are illustrated by polyethylene oxide condensates of C6-C12 alkylphenols; condensates of ethylene oxide with the reaction product of propylene oxide and ethylenediamine; long chain tertiary amine oxides; long chain tertiary phosphine oxides; long chain dialkyl sulfoxides; and the like. Suitable cationic surfactants are compounds containing positively charged amine or quaternary ammonium groups. Suitable amphoteric surfactants include derivatives of aliphatic quaternary ammonium, phosphonium and sulfonium compounds. One class of amphoteric surfactants are zwitterionic compounds such as betaines, sultaines and phosphobetaines. Illustrative of a betaine is cocoamidopropyl betaine. Another class of amphoteric surfactants are compounds containing an amine group and an anionic group such as carboxylate, sulfonate, sulfate, phosphate or phosphonate, as illustrated by sodium 3-dodecylaminopropionate and sodium 3-dodecylaminopropane sulfonate.

In a particular embodiment, the cosmetic composition comprises at least one of the following: sodium cocoamphoacetate, propylene glycol, sodium laureth sulfate, citric acid, sodium benzoate, salicyl acid.

The skin care compositions may include, apart from the above components, commonly known excipients including perfumes, pigments, colorants, dyes, waxes, masking agents, stabilizers, sunscreens, emulsifiers, medicaments, antiseptics, chelating agents, protectants, viscosifiers, vitamins, panthenol, ubiquinone Q10, hyaluronic acid, or any combinations thereof.

The skin care compositions described herein are useful for improving, maintaining and/or restoring skin microvascularization of the epidermal skin in a subject.

The term "subject" typically and preferably denotes humans, but may also encompass reference to non-human animals, preferably warm-blooded animals, even more preferably mammals, such as, e.g., non-human primates, rodents, canines, felines, equines, ovines, porcines, etc. The term "non-human animals" includes all vertebrates, e.g., mammals, such as non-human primates, particularly higher primates, sheep, dogs, rodents (e.g., mice or rats), guinea pigs, goats, pigs, cats, rabbits, cows, and non-mammals such as chickens, amphibians, reptiles etc. In certain embodiments, the subject is a non-human animal. In certain embodiments, the subject is a non-human mammal. In certain embodiments, the subject is a transgenic non-human animal or non-human mammal. In preferred embodiments, the subject is human. In other embodiments, the subject is an experimental animal or animal disease model. The term does not denote a particular age or sex, and includes inter alia newborns, children, adolescents, and adults including the elderly, whether male or female.

The cosmetic uses or methods as taught herein generally comprise administering to the skin of the subject a cosmetically effective amount of the skin care composition as taught herein, that is an amount sufficient to elicit the cosmetic effect relating to the prevention or reduction of skin aging in the subject, that is being sought by the cosmetician or beautician, in either a single or multiple doses.

The term "to administer" generally means to dispense or to apply, and typically includes both in vivo administration and ex vivo administration to a tissue, preferably in vivo administration. Generally, compositions may be administered systemically or locally. Given the nature of the present cosmetic treatments, and the nature of the active ingredient, the present compositions may be preferably configured for topical administration to the skin of the subject.

The term "topical administration" as generally used in the cosmetic and medical fields denotes the application of compositions directly to a part of the body. Particularly in the present case the term refers to topical administration onto the skin of a subject, more particularly onto the surface of the skin of the subject, and even more particularly onto a part, region or area of the surface of the subject's skin where the cosmetic or pharmacological effect is sought. Topical administration is typically not intended to and does not elicit any systemic effects.

When the compositions are applied to the skin, it is preferred that the amount of the composition applied to the skin is between 0.5 g and 2.0 g, more preferably between 0.5 g and 1.0 g. Stated differently, the amount of the composition may correspond to at least 1.0 x 10⁵ CFU, at least 1.0 x 10⁶ CFU, at least 1.0 x 10⁷ CFU, at least 1.5 x 10⁷ CFU, at least 2.0 x 10⁷ CFU or at least 2.5 x 10⁷ CFU.

The skin care composition of the present invention may be provided as ready-to-use composition which is suitable for direct topical administration to the skin. Such a composition may be provided in different forms, including, but not limited to, in the form of a gel, cream, lotion, ointment, past, soft, paste, suspension, solution, salve, wax, milk, emulsion, or the like. In such a composition, the (optionally lyophilized or spray-dried) live bacteria will be present in the admixture with other cosmetic or pharmaceutical excipients described elsewhere herein, such as emollients, fillers, and the like. Upon application of these compositions to the skin, the dried bacteria will be re-activated on the skin of the subject to which the product is applied. Growth of the re-activated bacteria from the skin care composition will positively influence the microbial flora on the skin of the subject.

The ready-to-use skin care compositions are preferably stable at room temperature for at least 1 week, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, at least 13 weeks, at least 14 weeks, at least 15 weeks, at least 16 weeks, at least 17 weeks, at least 18 weeks, at least 19 weeks, at least 20 weeks, at least 21 weeks, at least 22 weeks, at least 23 weeks, at least 24 weeks, at least 25 weeks, at least 26 weeks, at least 27 weeks, at least 28 weeks, at least 29 weeks, at least 30 weeks or more than 30 weeks. Stated differently, such compositions are preferably stable at room temperature for at least 1 month, at least 2 months, at least 3 months, at least 4 months, at least 5 months, at least 6 months or more than 6 months. As used herein, a composition is regarded as being stable if the reduction in the number of colony forming units present in the composition after storage is less than a 3 log reduction, preferably less than a 2 log reduction, and more preferably less than a 1 log reduction. Stated differently, a composition is regarded as being stable if the reduction in the number colony forming units present in the composition after storage is less than 1000-fold, preferably less than 100-fold, and more preferably less than 10-fold relative to the number of colony forming units in the composition before storage.

In some embodiments, the present invention provides a kit of parts as disclosed herein, in which the (optionally lyophilized or spray-dried) bacteria are spatially separated from the other components, e.g. the cosmetic components. For example, the kit of parts may be in the form of a packaging with two spatially separated chambers, wherein the first chamber contains the lyophilized or spray-dried bacteria, and the second chamber contains a composition comprising any of the other components. Prior to use, the contents of both chambers are mixed with each other, such as for example by a consumer, to provide a homogeneous skin care composition which is then applied to the skin. Alternatively, the composition comprising the one or more compounds can be applied to the skin first, followed by application of a composition comprising the one or more C. acnes strains. A kit of part assembly has the advantage that the bacteria can remain in lyophilized or spray-dried form until use which is associated with a particular high storage stability of the composition. In a kit of parts assembly, it is advantageous if the weight ratio of the bacteria in the first chamber to the composition comprising the one or more compounds in the second chamber is from 1:10, 1:20, 1:30, 1:40, 1:50, 1:60, 1:70, 1:80, 1:90, or 1:100. After mixing the contents of the both chambers, a skin care composition as described herein above is created. According to the present invention a kit of parts can be provided, for example, in a Lyo-Ject^{®} double-chamber syringe, in a V-LK^{®} double-chamber cartridge or in a dual-chamber system as described in WO 2018/077598. In another preferred embodiment, the (optionally lyophilized or spray-dried) bacteria in the first chamber may be suspended in a lipid or oil. This will significantly facilitate packaging and filling. In addition, the surrounding lipid or oil will protect the bacteria from premature dehydration. Preferably, the bacteria are suspended in elthylhexyl cocoate or dicaprylyl carbonate. The weight ratio of the bacteria to the oil or lipid preferably is between 1:1 and 1:2.

It is particularly preferred that the subject is a human. In some embodiments, in particular when the skin care composition is used to improve the appearance or youthful complexion of the skin, the subject may be one in whom signs of skin aging, such as wrinkles, lines, frown lines, loss of hydration, loss of elasticity, skin sagging, blemishes, and/or pigmentation changes ("age spots"), have started to manifest. In certain embodiments, the subject may be a human subject with an age of 40 or more years, such as 45 or more years, preferably 50 or more years, such as 55 or more years, more preferably 60 or more years, such as 65 or more years, for example 70 or more years, 75 or more years, or 80 or more years. A further aspect provides a method for stimulating or boosting the growth of at least one endogenous *C. acnes* strain on the skin of a subject, said method comprising the topical administration of a skin care composition comprising one or more compounds selected from the group consisting of an ester of a polyethylene glycol and a fatty acid, glycerol, sorbitol, and cetearyl sulphate to the skin of the subject. The aforementioned teachings are applicable mutatis mutandis to this aspect, in which a composition essentially as described throughout the specification, but leaving out the exogenous *C. acnes,* can be administered to modulate the growth of endogenous *C. acnes,* such as specifically certain clades or SLST types of endogenous *C. acnes.*

It is apparent that there have been provided in accordance with the invention products, methods, and uses that provide for substantial advantages as set forth above. While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and broad scope of the appended claims.

### BRIEF DESCRIPTION OF THE FIGURES

Figure 1 shows the results from the oxygen consumption measurements. The percentage of dissolved oxygen in HaCaT Keratinocyte cultures (dot, O₂^{Ker}), *C. acnes* cultures (crossed square, O₂^{C.acnes}) and mixed cultures of keratinocytes and *C. acnes* (triangle, O₂^{Ker.+C.acnes}) are shown. It can be seen that the oxygen consumed by mixed cultures of keratinocytes and *C. acnes* (O₂^{Ker.+C.acnes}) is higher than the mathematical sum of the oxygen consumed by keratinocytes and *C. acnes* in separate cultures (Σ(O₂^{Ker} + O₂^{C.acnes})).
Figure 2 shows the results from the viability measurements. Viability of non-infected controls cultured under aerobic and anaerobic conditions is expressed as percentage (%) of living cells (mean ± SD, n=2) and percentage of dead cells (mean ± SD, n=2).
Figure 3 shows the results of the metabolic activity assessment. Metabolic activity (test XTT, n=6) in the aerobically cultured infected keratinocytes cultures (Ker. + *C. acnes)* are expressed as percentage in relation to the control (Ker, white bar, non-infected aerobic cultures).
Figure 4 shows the results of the Gene Set Enrichment Analysis (GSEA). The figure shows a significantly enriched score when the control (non-infected co-cultures) is incubated under anaerobic conditions (adjusted *p*value 8×10⁻³).
Figure 5 shows the results of the RNAseq analysis. The figure shows that under anaerobic conditions, VEGFA is significantly upregulated in non-infected keratinocytes (FDR *p*value 8.43×10⁻⁹).
Figure 6 shows the results of the analysis of the VEGFA concentrations in supernatants of strain A1. The figure shows the means of concentrations (± SD, n=3) of VEGFA expressed in pg.ml-1 in supernatants of non-infected co-cultures and in co-cultures infected with *C*. *acnes* (+ *C. acnes)* that were incubated post-infection under aerobic (white bars) and anaerobic conditions (black bars).

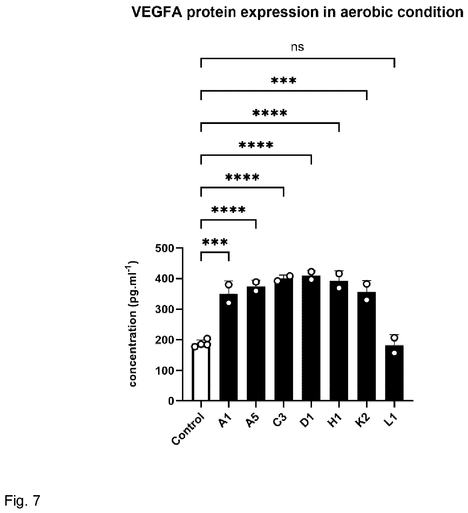

Figure 7 shows the results of the analysis of the VEGFA concentrations in supernatants of strains A1, A5, C3, D1, H1 and K2.

### EXAMPLES

The above aspects and embodiments are further supported by the following non-limiting examples.

### EXAMPLE 1: Oxygen consumption measurements

Keratinocytes and *C. acnes* cells were grown in separate cultures under the same conditions. Oxygen consumption in the cultures was measured and compared to oxygen consumption in mixed cultures of keratinocytes and *C. acnes.*

### HaCaT keratinocytes cultures

HaCaT cells (spontaneously immortalized human keratinocytes, HEK001, Cat. CRL-2404, ATCC) were cultured in Dulbecco's Modified Eagle Medium (Ref. DMEM, Cat. 41966052, Gibco) supplemented with 10% FBS (Foetal Bovine Serum, Cat. F7524, Sigma) in 75 cm² tissue culture flask (Cat. 734-2313, VWR). The cells were incubated for 6 days at 37°C, 5% CO₂. Once 80% confluence was reached, the keratinocyte cell layer was washed with Dulbecco's Phosphate Buffered Saline (D-PBS, 1x, (-) Calcium (-) Magnesium, Cat. 30-2200, ATCC) and subjected to trypsin treatment (2 ml, 37°C, 5% CO₂, 10 min Trypsin (0.25%) - EDTA (0.53mM) in HBSS, Cat. 30-2101, ATCC). The cells were subsequently stained with trypan blue (Trypan Blue 0.4%, Cat. EBT-001 NanoEnTek) and counted with an automated cell counter (EVE^{™} Automated Cell Counter, NanoEnTek). After the viability of the cells was assessed in the cell counter, they were transferred to a disposable polystyrene low-well multi dish (OxoDish^{®} OD24). 2 ml of culture (2.5×10⁵ cells/ml in DMEM) were used per well. The cells were re-incubated for 24 hours 37°C, 5% CO₂.

### Bacterial cultures

*Cutibacterium acnes* strain A1 was pre-cultured from glycerol stocks in 20 ml DMEM (Cat. 41966052, Gibco) in 25 cm² flask incubated anaerobically for 72 h (37°C, 90 rpm).

### Infection of the keratinocytes cultures

After 72 hours, aliquots were sampled to measure the concentration of the *C. acnes* cells using a predetermined method based on optical density (OD₆₀₀ₙₘ). In order to determine precisely the volume of bacterial culture to inject on top of the keratinocytes, the MOI (Multiplicity of Infection, number of bacterial cells infecting one keratinocyte) was determined. One well of the previously seeded keratinocytes was treated with trypsin and counted by using trypan blue (Trypan Blue 0.4%, Cat. EBT-001 NanoEnTek). Cell counting was performed in an automated cell counter (EVE^{™} Automated Cell Counter, NanoEnTek). In accordance with this updated cell count, a defined volume of bacterial cultures was diluted in fresh DMEM to reach MOI = 10 and was used to replace culture media in the OxoDish^{®} OD24 multi dish. The cells were re-incubated for 24 hours in aerobic (37°C, 5% CO₂) conditions.

### Oxygen consumption in infected HaCaT keratinocytes cultures

Oxygen consumption, expressed as percentage of dissolved oxygen in liquid DMEM media (Cat. 41966052, Gibco) was measured in OxoDish OD24 multi dish using a SDR Sensor Dish Reader (PreSens, Germany).

### Results

The results of the oxygen consumption measurement are shown in Figure 1. Measurements of oxygen consumption are expressed as percentage (%) of dissolved oxygen (y-axis) during 10 hours (x-axis), means ± SD of 4 biological replicates: n=4. The percentage of dissolved oxygen in HaCaT keratinocyte cultures (dot, O₂^{Ker}), *C. acnes* cultures grown under the same conditions as the keratinocytes (crossed square, O₂^{C.acnes}) and in mixed cultures of keratinocytes and *C. acnes* (triangle, O₂^{Ker.+C.acnes}) are shown. By computing the mathematical sum of oxygen consumed by the keratinocyte and the *C. acnes* culture (∑(O₂^{Ker} + O₂^{C.acnes}) and comparing it to the infected culture (O₂^{Ker.+C.acnes}), it can be seen that oxygen consumption is increased in the mixed culture. This means that oxygen consumption synergistically increases when culturing keratinocytes and *C. acnes* cells together in a mixed culture.

### EXAMPLE 2: VEGFA gene expression and VEGFA protein production in co-cultures of keratinocytes/sebocytes and C. acnes

The level of VEGFA gene expression and VEGFA protein expression was measured in keratinocytes/sebocytes that were co-cultured with *C. acnes* under aerobic and anaerobic conditions.

### HaCaT keratinocytes cultures

HaCaT cells (spontaneously immortalized human keratinocytes, HEK001, Cat. CRL-2404, ATCC) were cultured in Dulbecco's Modified Eagle Medium (Ref. DMEM, Cat. 41966052, Gibco) supplemented with 10% FBS (Foetal Bovine Serum, Cat. F7524, Sigma) in 75 cm² tissue culture flask (Cat. 734-2313, VWR). The cells were incubated for 6 days at 37°C, 5% CO₂. Once 80% confluence was reached, the keratinocyte cell layer was washed with Dulbecco's Phosphate Buffered Saline (D-PBS, 1x, (-) Calcium (-) Magnesium, Cat. 30-2200, ATCC) and subjected to trypsin treatment (2 ml, 37°C, 5% CO₂, 10 min Trypsin (0.25%) - EDTA (0.53mM) in HBSS, Cat. 30-2101, ATCC). The cells were subsequently stained with trypan blue (Trypan Blue 0.4%, Cat. EBT-001 NanoEnTek) and counted with an automated cell counter (EVE^{™} Automated Cell Counter, NanoEnTek). After the viability of the cells was assessed in the cell counter, they were transferred to Transwell inserts (Cat. 3460, Corning). 500 µl of culture (2.5×10⁵ cells/ml in DMEM) were used per insert. The cells were re-incubated for 24 hours 37°C, 5% CO₂.

### iPSC sebocytes cultures

iPSC sebocytes (induced pluripotent stem cells, Ref. Human iPSC-derived Sebocytes PCi-SEB_CAU, Phenocell, France) were propagated in PhenoCULT^{®}-SEB basal medium (Phenocell) supplemented with 1:1000 Supplement A (Phenocell) for 3 days at 37°C and 5% CO₂. The cultures were grown in 12 well plates (Cat. 3513. Corning) that had been previously coated with fibronectin (Cat. 33010-018, Gibco). On day 4, media was supplemented with Supplement M (Phenocell) to induce sebocytes maturation, and the cultures were incubated for 2 extra days at 37°C and 5% CO₂, until a final density of approximately 3×10⁵ cells/well was reached on day 6.

### Bacterial cultures

*Cutibacterium acnes* strain A1 was pre-cultured from glycerol stocks in 20 ml DMEM (Cat. 41966052, Gibco) in 25 cm² flask incubated anaerobically for 72 h (37°C, 90 rpm).

### Infection of the co-cultures

After 72 hours, aliquots were sampled to measure the concentration of the *C. acnes* cells using a predetermined method based on optical density (OD₆₀₀ₙₘ). In order to determine precisely the volume of bacterial culture to inject on top of the keratinocytes, the MOI (Multiplicity of Infection, number of bacterial cells infecting one keratinocyte) was determined. One Transwell insert of the previously seeded keratinocytes was treated with trypsin and counted by using trypan blue (Trypan Blue 0.4%, Cat. EBT-001 NanoEnTek). Cell counting was performed in an automated cell counter (EVE^{™} Automated Cell Counter, NanoEnTek). In accordance with this updated cell count, a defined volume of bacterial cultures was diluted in fresh DMEM to reach MOI = 10 and was used to replace culture media in the Transwell inserts. Post-infection, co-cultures were assembled by transferring Transwell inserts (keratinocytes) to the wells containing the iPSC sebocytes cultures. Newly infected Transwell inserts of keratinocytes co-cultured with iPSC Sebocytes were re-incubated for 24 hours under aerobic (37°C, 5% CO₂) and anaerobic (37°C, 0% CO₂) conditions.

### Analysis of keratinocyte viability, metabolic activity, and cytokines

After 24 hours, the culture media were gently pipetted up and down for homogenization and transferred on ice (1ml). The media were centrifuged (5000 rpm, 5min, 4°C), and the supernatants were stored at -80°C for further analysis. After collection of the supernatants, cell proliferation was assessed by using trypan blue (Trypan Blue 0.4%, Cat. EBT-001 NanoEnTek) and an automated cell counter (EVE^{™} Automated Cell Counter, NanoEnTek). Viability of the controls (non-infected cultures incubated under aerobic and anaerobic conditions) was assessed using a live/dead kit (Cat. R37601, ThermoFisher). Metabolic activity of aerobically cultured infected keratinocytes cell layers was assessed using the XTT method and expressed as % compared to the controls (Cat. 11465015001, Merck).

### VEGFA gene expression (RNAseq) analysis

Keratinocyte cell layers were scraped (Cat. CLS3010, Merck) and RNA extracted using the kit QIAzol Lysis Reagent (Cat. 79306, Qiagen) following the manufacturer's instructions.

After extraction, the RNA samples were stored and shipped at -80°C before sequencing (Macrogen, Europe). RNAseq, analysis with the raw counts was done with R (v4.1.2). Gene Set Enrichment Analysis (GSEA) was performed with fgsea (v1.20), the gene sets were retrieved from the Molecular Signatures Database (MSigDB) with msigdbr (v7.5.1). For differential expression analysis, edgeR (v3.36.0) was used.

### Protein excretion and VEGFA quantification analysis

The Human Cytokine/Chemokine Discovery Assay Array (Eve Technologies Corporation, Canada) was used to quantify VEGFA in the previously collected supernatants. VEGFA amounts were expressed as pg.ml⁻¹. VEGFA concentrations were expressed as mean ± SD (n=3) for each conditions and statistical analysis was performed in GraphPad Prism). Statistical significance was calculated by comparing the samples to the aerobic control with ordinary one way ANOVA, ****: p < 0.0001, ***: p < 0.001, **: p < 0.01, *: p < 0.05.

### Results

The results of the viability assessment are shown in Figure 2. The figure shows the percentage (%) of living cells (means ± SD, n=2) and dead cells (means ± SD, n=2) in the non-infected keratinocytes cultures (controls) incubated under aerobic (37°C, 5% CO₂) and anaerobic (37°C, 0% O₂) conditions. Considering that the total number of cells per well measured was equivalent in both the aerobic and anaerobic cultures (1,400,000 ± 141421 aerobic and 1,350,000 ± 70711 anaerobic cells/well respectively, n=2), there was no significant viability difference in the cell layers under these conditions. Furthermore, this equal total number of cells per well was significantly higher than the seeding density. This proves that hypoxia neither induces cell death nor arrests cell division in the different keratinocyte cultures. Therefore, gene and protein expression can be analysed and compared under these conditions.

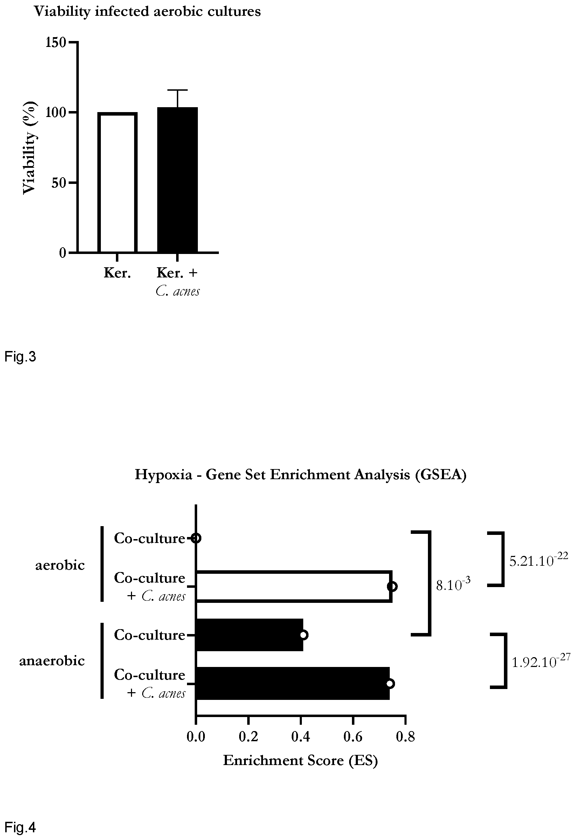

The results of the metabolic activity assessment are shown in Figure 3. Metabolic activity (test XTT, n=6) in the aerobically cultured infected keratinocytes cultures (Ker. + *C. acnes)* are expressed as percentage to the control (Ker., white bar, non-infected aerobic cultures (37°C, 5% CO₂). There is no difference between the controls and the infected cultures. This proves that the observed hypoxia in presence of oxygen neither induces cell death nor arrests cell division in the different keratinocyte cultures. Also, no aberrant proliferation was found in the different keratinocyte cultures. Therefore, gene and protein expression can be analysed and compared under these conditions.

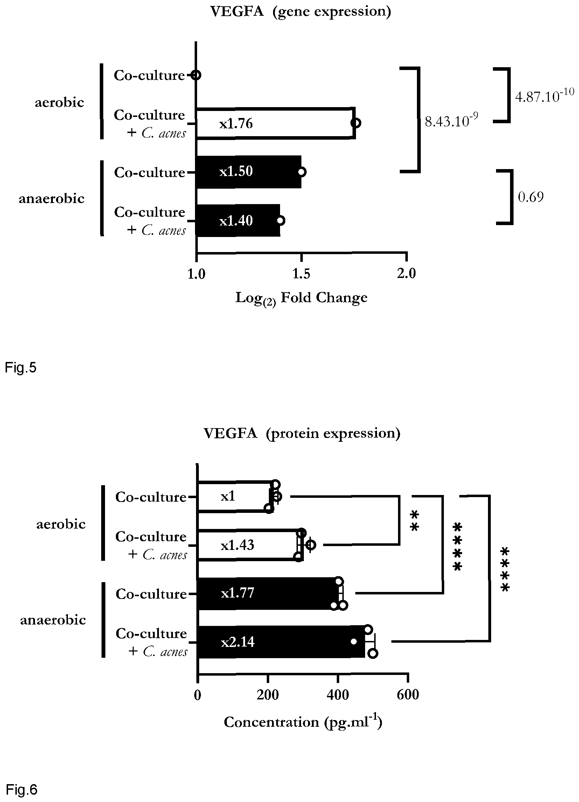

The results of the gene expression analysis are shown in Figure 4 and 5. Gene Set Enrichment Analysis (GSEA, Hallmark pathways, Molecular Signatures Database (MSigDB) with msigdbr (v7.5.1)) of the genes sets (n=3 per conditions) compared to the aerobic control (non-infected Keratinocytes cultures). Hallmark pathway "Hypoxia" (size=171) shows a significantly enriched score when the controls (non-infected co-cultures) are incubated under anaerobic conditions (adjusted *p*value 8×10⁻³). This validates the positive controls for hypoxia condition. See Figure 4. The hypoxia is maintained in infected anaerobic cultures and even increases when considering the very low adjusted *p*value (1.92×10⁻²⁷). In the aerobic co-cultures infected with *C. acnes,* a high significantly induced hypoxia is observed (adjusted *p*value 5.21×10⁻²²). That unambiguously demonstrates that in the presence of oxygen the keratinocyte and *C. acnes* cells shape an anaerobic niche turning the physiological state to hypoxia. Figure 5 shows that VEGFA is differently expressed (edgeR (v3.36.0)) when compared to non-infected aerobic keratinocyte cultures (n=3 per conditions). Under anaerobic conditions, VEGFA is significantly upregulated in non-infected keratinocytes (FDR *p*value 8.43×10⁻⁹), validating the positive control of VEGFA overexpression under hypoxia. This remains unchanged when the cultures are infected with *C. acnes* under anaerobic conditions. In aerobically infected co-cultures (white bar), the keratinocyte and C. acnes cells synergistically decrease oxygen to promote hypoxia which induces the upregulation of VEGFA. Log₂ Fold Change = 1.76, FDR *p*value 4.87×10⁻¹⁰.

The results of the analysis of the VEGFA concentrations in supernatants of strain A1 are shown in Figure 6. The figure shows the means of the concentrations (± SD, n=3) of VEGFA (pg.ml⁻¹) in supernatants of non-infected co-cultures and in co-cultures infected with *C*. *acnes* (+ *C. acnes*) which were incubated post-infection under aerobic (white bars) and anaerobic conditions (black bars). For statistical analysis, ordinary one-way ANOVA multiple comparisons with the mean of aerobic co-culture concentrations were performed for all the other conditions, **: *p*value < 0.01, ****: *p*value < 0.0001. Labels in the bars show the fold change (FC). Comparison of aerobic versus anaerobic non-infected co-culture show that hypoxia induces both gene expression (see Figure 5) and VEGFA protein excretion (this figure, FC = 1.77), validating the positive control. The statistically significant higher concentration of VEGFA in infected anaerobic co-cultures is in the same magnitude (FC = 2.14). The statistically significant higher concentration of VEGFA in aerobic co-cultures proves that *C. acnes* triggers its expression and excretion similarly in anaerobicity or aerobicity, by decreasing synergistically oxygen when present.

Similar results in terms of VEGFA concentrations were observed in in supernatants of strains A1, A5, C3, D1, H1 and K2.

From the above results, it can be concluded that when cultured under aerobic conditions, keratinocytes express VEGFA, both on the gene and protein level (basal activity). When cultured under anaerobic conditions (positive control) hypoxia is increased and keratinocytes overexpress VEGFA, both on the gene and protein level. When incubated under anaerobic conditions in the presence of *C*. *acnes,* hypoxia and VEGFA production are maintained. When incubated under aerobic conditions in the presence of *C*. *acnes,* hypoxia is increased and both VEGFA gene expression and VEGFA protein production are activated. *C. acnes* decreases synergistically the oxygen tension at the surface of aerobic keratinocyte cultures leading to the overexpression of VEGFA in aerobic condition similarly to anaerobic cultures.

### REFERENCES

Fitz-Gibbon et al. 2013. Propionibactium acnes strain populations in the human skin microbiome associated with acne. J Invest Dermatol 133(9).
Holmberg et al. 2009. Biofilm formation by Propionibacterium acnes is a characteristic of invasive isolates. Clin Microbiol Infect 15:787-95.
Johnson and Cummins, 1972. Cell wall composition and deoxyribonucleic acid similarities among the anaerobic coryneforms, classical proprionibacteria and strains of Arachnia pro-pionica. J Bacteriol. 109(3): 1047-66.
Lodes et al., 2006. Variable expression of immunoreactive surface proteins of Propionibacterium acnes. Microbiology 152:3667-3681.
Lomholt and Kilian, 2010. Population and genetic analysis of Propionibacterium acnes identifies a subpopulation and epidemic clones associated with acne. Plos One 5(8) e12277.
Mc Dowell at al., 2005. Propionibacterium acnes types I and II represent phylogenetically distinct groups. J Clin Microbiol 43(1):326-334.
McDowell et al., 2008. A new phylogenetic group of Propionibacterium acnes. J Med Microbiol 57:218-224.
McDowell et al., 2012. An expanded multilocus sequence typing scheme for propionibacteium acnes: investigation of 'pathogenic', 'commensal' and antibiotic resistant strains. Plos One 7 (7) e41480.
Resvani et al., 2011. HIF-1alpha in epidermis: oxygen sensing, cutaneous angiogenesis, cancer, and non-cancer disorders. J Invest Dermatology 2011, 131 (9).
Scholz et al., 2014. A novel high-resolution single locus sequence typing scheme for mixed populations of Propionibacterium acnes in vivo. Plos One, 9(8) e104199.
Valanne at al. 2005. CAMP factor homologues in Propionibacterium acnes: a new protein family differentially expressed by types I and II. Microbiology 151:1369-1379.
Wang et al., 2003. Oxygen partial pressure in outer layers of skin in human finger nail folds. J Physiol 2003, 549 (Pt3).

## Claims

1. Skin care composition comprising live bacteria of at least one C. acnes strain for use in a method of improving, maintaining and/or restoring skin microvascularization.

2. Cosmetic use of a skin care composition comprising live bacteria of at least one C. acnes strain for improving, maintaining and/or restoring skin microvascularization.

3. Cosmetic method for improving, maintaining and/or restoring microvascularization in a subject, said method comprising the topical administration of a skin care composition comprising live bacteria of at least one C. acnes strain to the skin of the subject.

4. Skin care composition for use in a method of claim 1, cosmetic use of claim 2 or method of claim 3, wherein the at least one C. acnes strain, upon administration, contributes to creating an anaerobic microenvironment on the surface of the epidermal skin.

5. Skin care composition for use, cosmetic use or method of claim 4, wherein said anaerobic microenvironment on the surface of the epidermal skin is **characterized by** a partial oxygen pressure (POz) in the epidermal skin of less than 60 mm Hg, preferably less than 40 mm Hg.

6. Skin care composition for use, cosmetic use or method of any of the preceding claims, wherein said live bacteria are lyophilised or spray-dried.

7. Skin care composition for use, cosmetic use or method of claim 6, wherein said live bacteria are lyophilised.

8. Skin care composition for use, cosmetic use or method of any of the preceding claims, wherein each of said at least one C. acnes strain is independently selected from the group consisting of Phylotype clades I, II, and II.

9. Skin care composition for use, cosmetic use or method of any of the preceding claims, wherein each of said at least one C. acnes strain is independently selected from the group consisting of the single-locus sequence typing (SLST) type strains A1, D1, A5, C1, C3, H1, H2, H3, K1, K2, K4, K6, K8, K9, L1, and F4.

10. Skin care composition for use, cosmetic use or method of claim 9, wherein each of said at least one C. acnes strain is independently selected from the group consisting of the SLST type strain A1, D1 and H1.

11. Skin care composition for use, cosmetic use or method of claim 10, wherein said at least one C. acnes strain is SLST type strain H1.

12. Skin care composition for use, cosmetic use or method of any of the preceding claims, wherein the skin care composition comprises at least 10⁴ colony-forming units per ml (CFU/ml) of each live C. acnes bacterial strain, preferably about 10⁴-10⁹ CFU/ml of each live C. acnes bacterial strain, relative to the volume of the skin care composition.

13. Skin care composition for use, cosmetic use or method of any of the preceding claims, wherein the skin is human skin.

14. Skin care composition for use, cosmetic use or method of any of the preceding claims, wherein the skin care composition is a gel, cream, ointment, lotion, drops, spray including aerosol spray, foam or powder, optionally wherein the composition is comprised by a mask, pad, patch, a two-chamber device, or make-up.

15. Skin care composition for use, cosmetic use or method of any of the preceding claims, wherein at least one C. acnes strain, upon administration to the surface of the epidermal skin, increases the expression of Vascular Endothelial Growth Factor A (VEGFA) by skin keratinocytes.
